# EUROPEAN PATENT SPECIFICATION

(11) **EP 1 674 399 B1**
(45) Date of publication and mention of the grant of the patent: **22.11.2006**
(21) Application number: 04030418.0
(22) Date of filing: 22.12.2004
(51) Int. Cl.: B65D 1/02, B65D 65/38, A61K 31/573, B32B 27/20

(54) **Use of a container made of a plastic material containing an inorganic additive**
Verwendung eines Behälters aus Plastik mit anorganischem Zusatzstoff
Emploi d'un conteneur à matière plastique incluant un additif inorganique

(43) Date of publication of application: 28.06.2006
(73) Proprietor: Mattern, Udo, 6370 Stans (CH)
(72) Inventor: Mattern, Claudia, 6370 Stans (CH)
(74) Representative: Winkler, Andreas Fritz Ernst

(56) References cited:
- EP-A- 0 084 922
- GB-A- 761 618
- US-A- 3 923 190
- US-A- 5 948 492
- US-A1- 2002 114 933
- US-B1- 6 669 879

## Description

The present invention relates to the use of a container of an inorganic additive containing plastic material.

### Decription of the Related Art

Plastic containers are readily used for pharmaceutical preparations. However it is known that, due to their character, there are some limitations. Thus to suppress the reactivity of containers from polyethylene or polypropylene and their copolymers / blends towards certain chemicals several methods are used: plasticizers are avoided which would increase the motility of the chain molecules, polymers of higher density or polyolefin blends (e.g. polypropylene/polyacrylate) are used, the wall thickness is increased or the containers are wrapped (e.g. aluminium foil) or sealed (e.g. fluorination, silicone).

US patent 4,123,417 (Finberg, 1978) claims that the toughness of LDPE can be increased by a blend comprising low density polyethylene containing an amorphous ethylene-propylene copolymer having a certain amount of crystallinity and a specified ethylene content.

US patent 4,546,882 (Hsu et al., 1985) claims a multiple layer package for oil-containing products comprising an oil barrier layer from nylon or ethylene vinyl alcohol.

US patent 5,500,261 (Takei et al., 1996) claims an oil resistant container comprising a blended resin composition having specified glass-transition temperatures.

US patent 6,800,363 (Su et al., 2004) claims a film that does not distort in the presence of food oils using a polyolefin multilayer film having a skin layer from oil-absorbing porous particles (calcium carbonate, silicone dioxide, amorphous silica, sodium aluminosilicate, activated charcoal) and a metallized layer.

US patent 6,815,506 (Takashima et al., 2004) claims an oil-resistant thermoplastic elastomer composition comprising a propylene resin, an unsaturated group-containing acrylic rubber and an inorganic filler for rubber compositions, preferred silica.

Also other additives are usual to improve the properties of plastics. Of high importance are pigments and ultraviolet stabilizers (organic and inorganic pigments, dyes, benzophenone, hindered amines etc.). These cover a broad spectrum of requirements, such as heat stability, fastness to light and weathering, where titanium dioxide (TiO₂) is most common in pharmaceuticals. TiO₂ is an inert substance known for its broad spectrum of UV-absorption and non-migration (movement into the drug formulation).

### Summary of the invention

It is an object of the invention to provide an alternative use of containers made of an additive containing plastic material, which containers contain an oil, fat and/or wax containing formulation.

This object is achieved by an use of a container, made of an additive containing plastic material, for reducing physical/chemical interaction between the container and an oil, fat and/or wax containing formulation contained therein.

Preferably, the physical/chemical interaction is an adsorption of the formulation to the plastic material.

The inorganic additive is titanium dioxide (TiO₂), surface-treated titanium dioxide, or a mixture thereof.

The additive is present in the plastic material in an amount between 0.1 and 10 % by weight, more preferably between 0.1 and 5 % by weight, and most preferably about 2 % by weight, based on the weight of the plastic material.

The plastic material may comprise polyolefin.

Preferably, the polyolefin is selected from the group of polyethylene, polypropylene, copolymers of ethylene and propylene, or a mixture thereof.

More preferably, the plastic material comprises low density polyethylene (LDPE).

The plastic material may be suitable for extrusion blow molding.

The formulation comprises at least one steroid hormone dissolved or suspended in oil, fat and/or wax.

More preferred, the steroid hormone is a sexual hormone drug, preferably testosterone, and the formulation further comprises at least one lipophilic or partially lipophilic carrier; and a compound or a mixture of compounds having surface tension decreasing activity, in an amount effective for in situ generation of an emulsion upon contact of the formulation with water.

Finally, the formulation is preferably for nasal application, preferably to a mammalian.

A preferred low density polyethylene is for example Lupolen® 1840 H. Further, a preferred formulation may be the one which is disclosed in EP 03025769.5.

Surprisingly, it was found that a container of an inorganic additive containing plastic material may be advantageously utilized for keeping oil, fat and/wax containing formulations, for example oily formulations of steroid hormones, in that the use of such a container will reduce physical-chemical interactions of the container and the formulation, especially the adsorption of the formulation to the plastic material.

Surprisingly, the inventor has found that TiO₂ can also be used for a purpose for which it was not intended to be used so far: By adding it to plastic packaging material the physical-chemical interaction of certain oily formulations with the container, restricting its use, can be prevented.

The approaches actually made dealing with oil - plastic interaction did not use inorganic additive auxiliary agents nor a possibility was described for protecting a corresponding steroid hormone containing formulation from adsorption to plastic.

### Detailed description of the invention

In nasal application forms the suitability of the device for administration is of major importance. This applies to improving patient's compliance by convenient administration. But this also applies to pharmaceutical necessities such as the uniformity of emitted dose and the compatibility of the formulation with the primary packaging material. In pharmaceutical applications it is essential to use inert material for primary packaging; the galenical formulation, the active ingredient and the excipients, should not adversely be influenced by any interaction.

In principle there are two materials and two types for packaging of nasal formulations: glass vs. plastic and multiple-dose vs. unit-dose containers. The main advantages of plastic materials are their flexibility allowing for a wide range of designs, low weight, shatter resistance, and easy handling. Especially suitable for nasal application are unit-dose containers from plastic because of their small size, because no pump mechanism is necessary nor the addition of preservatives to the product formulation.

As starting material for such plastic containers polyethylene or polypropylene and their copolymers are used. Possible drawbacks in respect of their use are the oxygen permeability, poor UV resistance and, due to the nonpolar character, degree of crystallinity and molar mass, the poor resistance to some chemicals.

Thus polyethylene and polypropylene are not generally resistant to aliphatic and aromatic hydrocarbons and their halogen derivatives as well as to low-volatility substances such as fats, oils and waxes. Incompatibilities which can be seen are adsorption of the chemicals to the plastic, diffusion and swelling by the chemicals, or even dissolution in the chemicals.

On the other hand hydrocarbon derivatives such as steroid hormones are readily formulated using oil as carrier to increase their solubility and time of action. To avoid stability problems caused by the primary packaging these oily formulations - mostly injectables - usually are filled into glass devices. This kind of packaging however is not suitable for all application forms, e.g. not for oily formulations for nasal application. In concern of multi-dose devices the reason is that, although the bottle might be from glass, there are always parts of the device, such as the pump, which are from plastic material. In concern of unit-dose devices the reason is that these, at least in the case of viscous formulations which have to be squeezed, cannot be made from glass but moulded from plastics, mostly by the blow-fill-seal technology.

As an example for the aforementioned considerations in table 1 are shown the results of tests investigating the stability of formulations containing the steroid hormone testosterone in containers of different material.

**Table 1 Stability of formulations containing testosterone in containers of different material**

| **Primary packaging material** | **Formulation** | **Remaining drug after storage (%)** |
|---|---|---|
| LDPE | Oil-based | ≈ 30% |
| PP | Oil-based | ≈ 50% |
| Glass | Oil-based | ≈ 80% |
| Glass | Methanolic | 100% |
| LDPE + TiO₂ | Oil-based | 100% |

The term "remaining drug after storage" is the amount of testosterone remaining in the formulation after storage for 22 hours. The remaining drug was measured by HPLC technique.

It is obvious that there is a complex interaction of the drug with the oily formulation and of the oily formulation with the primary packaging material. For clinical-pharmaceutical reasons however the oil-based formulation and a unit-dose device for packaging was preferred. Thus some effort was made by the applicant using complicated procedures to solve this problem. Surprisingly however after adding titanium dioxide to the plastic material by this simple step it was possible to increase the shelf-life of the pharmaceutical formulation.

The features disclosed in the foregoing description and in the claims may, both separately and in any combination thereof, be material for realizing the invention in diverse forms thereof.

## Claims

1. Use of a container, made of an inorganic additive containing plastic material, for reducing physical/chemical interaction between the container and an oil, fat and/or wax containing formulation contained therein, **characterized in that** the inorganic additive is titanium dioxide and the formulation comprises at least one steroid hormone dissolved or suspended in oil, fat and/or wax, wherein the additive is present in the plastic material in an amount between 0.1 and 10 % by weight, based on the weight of the plastic material.

2. Use of the container according to claim 1 wherein the physical/chemical interaction is an adsorption of the formulation to the plastic material.

3. Use according to claim 1 or 2, wherein the titanium dioxide is surface-treated titanium dioxide.

4. Use according to any of the preceding claims, wherein the additive is present in an amount of between 0.1 and 5 % by weight, and most preferably about 2 % by weight, based on the weight of the plastic material.

5. Use according to any of the preceding claims, wherein the plastic material comprises polyolefin.

6. Use according to claim 5, wherein the polyolefin is selected from the group of polyethylene, polypropylene, copolymers of ethylene and propylene, or a mixture thereof.

7. Use according to claim 6, wherein the plastic material comprises low density polyethylene (LDPE).

8. Use according to any of the preceding claims, wherein the plastic material is suitable for extrusion blow molding.

9. Use according to any of the preceding claims, wherein the steroid hormone is a sexual hormone drug, preferably testosterone, and the formulation further comprises at least one lipophilic or partially lipophilic carrier; and a compound or a mixture of compounds having surface tension decreasing activity, in an amount effective for in situ generation of an emulsion upon contact of the formulation with water.

10. Use according to any of the preceding claims, wherein the formulation is for nasal application, preferably to a mammalian.

## Patentansprüche

1. Verwendung eines Behälters, hergestellt aus einem ein anorganisches Additiv enthaltenden Kunststoffmaterial, zur Reduzierung physikalischer/chemischer Wechselwirkung zwischen dem Behälter und einer ein Öl, ein Fett und/oder ein Wachs enthaltenden Zubereitung, die darin enthalten ist, **dadurch gekennzeichnet, daß** das anorganische Additiv Titandioxid ist und die Zubereitung wenigstens ein Steroidhormon aufgelöst oder suspendiert in Öl, Fett und/oder Wachs umfaßt, wobei das Additiv im Kunststoffmaterial in einer Menge zwischen 0,1 und 10 Gew.-%, basierend auf dem Gewicht des Kunststoffmaterials, vorhanden ist.

2. Verwendung des Behälters nach Anspruch 1, wobei die physikalische/chemische Wechselwirkung eine Adsorption der Zubereitung am Kunststoffmaterial ist.

3. Verwendung nach Anspruch 1 oder 2, wobei das Titandioxid oberflächenbehandeltes Titandioxid ist.

4. Verwendung nach einem der vorangehenden Ansprüche, wobei das Additiv in einer Menge zwischen 0,1 und 5 Gew.-% und am bevorzugtesten etwa 2 Gew.-%, basierend auf dem Gewicht des Kunststoffmaterials, vorhanden ist.

5. Verwendung nach einem der vorangehenden Ansprüche, wobei das Kunststoffmaterial Polyolefin umfaßt.

6. Verwendung nach Anspruch 5, wobei das Polyolefin ausgewählt wird aus der Gruppe aus Polyethylen, Polypropylen, Copolymeren aus Ethylen und Propylen oder einer Mischung derselben.

7. Verwendung nach Anspruch 6, wobei das Kunststoffmaterial Polyethylen niedriger Dichte (LDPE) umfaßt.

8. Verwendung nach einem der vorangehenden Ansprüche, wobei das Kunststoffmaterial zum Extrusionsblasformen geeignet ist.

9. Verwendung nach einem der vorangehenden Ansprüche, wobei das Steroidhormon ein Sexualhormonarzneimittel, bevorzugt Testosteron, ist und die Zubereitung ferner wenigstens einen lipophilen oder teilweise lipophilen Träger umfaßt; und eine Verbindung oder eine Mischung von Verbindungen mit Oberflächenspannungsabsenkender Aktivität, in einer Menge, die für eine in situ-Erzeugung einer Emulsion bei Kontakt der Zubereitung mit Wasser effektiv ist.

10. Verwendung nach einem der vorangehenden Ansprüche, wobei die Zubereitung zur nasalen Anwendung, bevorzugt an ein Säugetier, ist.

## Revendications

1. Utilisation d'un conteneur, constitué d'un additif inorganique contenant un matériau plastique, afin de réduire l'interaction physico-chimique entre le conteneur et une huile, une graisse et/ou une cire contenant une préparation contenue dans celui-ci, **caractérisé en ce que** l'additif inorganique est du dioxyde de titane et la préparation comprend au moins un stéroïde hormonal dissout ou en suspension dans l'huile, la graisse et/ou la cire, dans laquelle l'additif est présent dans le matériau plastique en une quantité comprise entre 0,1 et 10 % en poids, sur la base du poids du matériau plastique.

2. Utilisation du conteneur selon la revendication 1 dans laquelle l'interaction physico-chimique est une adsorption de la préparation vers le matériau plastique.

3. Utilisation selon la revendication 1 ou 2, dans laquelle le bioxyde de titane est du bioxyde de titane traité en surface.

4. Utilisation selon l'une quelconque des revendications précédentes, dans laquelle l'additif est présent en une quantité comprise entre 0,1 et 5 % en poids, et de façon préférée entre toutes environ 2 % en poids, sur la base du poids du matériau plastique.

5. Utilisation selon l'une quelconque des revendications précédentes, dans laquelle le matériau plastique comprend une polyoléfine.

6. Utilisation selon la revendication 5, dans laquelle la polyoléfine est choisie dans le groupe du polyéthylène, du polypropylène, des copolymères d'éthylène et de propylène, ou un mélange de ceux-ci.

7. Utilisation selon la revendication 6, dans laquelle le matériau plastique comprend un polyéthylène de faible densité (LDPE).

8. Utilisation selon l'une quelconque des revendications précédentes, dans laquelle le matériau plastique est adapté au moulage par extrusion soufflage.

9. Utilisation selon l'une quelconque des revendications précédentes, dans laquelle le stéroïde hormonal est un médicament à base d'hormone sexuelle, de préférence de la testostérone, et la préparation comprend en outre au moins un vecteur lipophilique ou partiellement lipophilique ; et un composé ou un mélange de composés ayant une activité de réduction de la tension superficielle, en une quantité efficace pour la génération in situ d'une émulsion lors du contact de la formulation avec l'eau.

10. Utilisation selon l'une quelconque des revendications précédentes, dans laquelle la préparation est destinée à une application nasale, de préférence à un mammifère.
